# EUROPEAN PATENT SPECIFICATION

(11) **EP 1 722 647 B1**
(45) Date of publication and mention of the grant of the patent: **22.04.2009**
(21) Application number: 05738083.4
(22) Date of filing: 09.03.2005
(51) Int. Cl.: A23L 2/02, A23L 2/84

(54) **PREBIOTIC USE OF FRUITS AND FRUIT JUICES IN THE PROMOTION OF BENEFICIAL GUT MICROFLORA**
PRÄBIOTISCHE VERWENDUNG VON FRÜCHTEN UND FRUCHTSÄFTEN BEI DER FÖRDERUNG EINER GÜNSTIGEN DARMFLORA
UTILISATION PREBIOTIQUE DE FRUITS ET DE JUS DE FRUITS AFIN DE PROMOUVOIR LE DEVELOPPEMENT BENEFIQUE DE LA MICROFLORE INTESTINALE

(30) Priority: 11.03.2004 GB 0405540
(43) Date of publication of application: 22.11.2006
(73) Proprietor: GLAXO GROUP LIMITED, Greenford, Middlesex UB6 ONN (GB)
(72) Inventor: CLIFFORD, Mike, Guildford Surrey GU2 7XH (GB); GIBSON, Glenn, University of Reading, Reading Berkshire RG6 6AP (GB); HU, Henglong, GlaxoSmithKline, Coleford Gloucestershire GL16 8JB (GB); RODIG-PENMAN, Andrea, Gloucestershire GL16 8 JB (GB)
(74) Representative: Giddings, Peter John
(86) International application number: PCT/EP2005/002701
(87) International publication number: WO 2005/092127

(56) References cited:
- MCMILLAN: "To your Health" SOFT DRINKS INTERNATIONAL, 28 July 2003 (2003-07-28), XP008048937
- PATENT ABSTRACTS OF JAPAN vol. 2000, no. 05, 14 September 2000 (2000-09-14) & JP 2000 032909 A (BIO HEALTH:KK), 2 February 2000 (2000-02-02)
- DATABASE WPI Section Ch, Week 200011 Derwent Publications Ltd., London, GB; Class B04, AN 2000-125456 XP002332963 & RU 2 120 295 C1 (GLUBOKII G I) 20 October 1998 (1998-10-20)
- DATABASE WPI Section Ch, Week 200254 Derwent Publications Ltd., London, GB; Class D13, AN 2002-501289 XP002332962 & CN 1 344 502 A (WANG Y) 17 April 2002 (2002-04-17)
- DATABASE WPI Section Ch, Week 200512 Derwent Publications Ltd., London, GB; Class D13, AN 2005-104910 XP002333067 & JP 2005 013197 A (BIOENERGY YG) 20 January 2005 (2005-01-20)

## Description

The present invention relates to the prebiotic effects of dark fruit and dark fruit juice and their use in the promotion of growth of beneficial gut microflora.

The human gut microflora comprises more than 500 different species of bacteria that have a great metabolic impact upon human health (Roberfroid et al., Nutrition Reviews 53: 127-130, 1995; Steer et al., Nutrition Research Reviews 13: 229-254, 2000). One important function of the gut microflora, from the host's point of view, is to prevent colonization of potentially pathogenic microorganisms by outcompeting invading pathogens for ecological niches and metabolic substrates. Microbiological metabolism in the gut also serves as an important source of energy for coloncytes and as a source of B vitamins and vitamin K. The gut microflora act as an important modulator of the immune system, not only educating the naive infant immune system but also serving as an important source of non-inflammatory immune stimulation throughout life in healthy individuals.

The gut micorflora can be divided into potentially deleterious and potentially health-promoting species. For example, some *Clostridium spp*., and proteolytic *Bacteriodes spp*. are considered potentially harmful because of their association with certain acute and chronic gastrointestinal complaints. Their metabolic end products are toxic and can cause cellular destruction in the bowel. Moreover, some products may enter the bloodstream and exert negative effects systemically. On the other hand, *Bifidobacterium spp*., and the lactic acid bacteria, particularly *Lactobacillus spp*. are considered to play an important role in a healthy gut ecosystem through their antagonistic activities towards potential pathogens, immunomodulatory activities, production of short chain fatty acids and reduction of microflora associated enzyme activities involved in the production of carcinogens and gentoxins (Gibson and Roberfroid, Journal of Nutrition 125, 1401-1412 1995; Yaeshima International Journal of Food Microbiology 30: 303-31 1996).

There is currently much interest in the concept of actively managing the colonic microflora with the aim of improving host health. This has been traditionally attempted by the consumption of live microbial food supplements known as probiotics. A probiotic has been defined as 'a living microbial food ingredient that is beneficial to health' (Diplock et al., British Journal of Nutrition, 1999). The most well studied and commercially used probiotics are *lactobacilli* and *bifidobacteria*. Examples of products containing such probiotics are yoghurts and dairy-based drinks such as Actimel^{™} (commercially available in the United Kingdom).

An alternative approach is the consumption of other types of food ingredients known as prebiotics. A prebiotic has been defined as 'a nondigestible food ingredient that beneficially affects the host by selectively stimulating the growth and/or activity of one or a limited number of bacteria in the colon and thus improves host health' (Gibson and Roberfroid, Journal of Nutrition 125, 1401-1412 1995).

According to Collins & Gibson (American Journal of Clinical Nutrition 1999; 69 (suppl)), for a food ingredient to be classified as a prebiotic, it should:
1. be neither hydrolysed nor absorbed in the upper part of the gastro-intestinal tract;
2. be a selective substrate for one or a limited number of potentially beneficial commensal bacteria in the colon, thus stimulating the bacteria to grow, become metabolically activated, or both; and
3. be able as a consequence to alter the colonic microflora toward a more healthy composition. (Collins & Gibson,).

As such, they fortify indigenous gut flora components that are thought to be of benefit.
Known prebiotics are dietary soluble fibres such as inulin, lactulose, fructo-oligosaccharides and galacto-oligosaceharides, which are able to survive the digestion and selectively stimulate the beneficial members of the gut microflora, such as bifidobacteria, in the colon (Gibson et al., New Developments in Functional Foods. Oxford: Chandos Publishing Limited 2000).

JPA 2005-013197 relates to a juice containing mainly aloe vera and a blend of fruits, such as papya and dragon fruit, having a prebiotic effect.

US patent application US 2003/0060445 describes nutritional formulations containing prebiotic substances, the substances being a combination of oligofructose and sialyllactose.

WO 02/091833 describes pharmaceutical compositions comprising a probiotic, a prebiotic and an ammoniaphilic urea-degrading microorganism with high alkali pH stability and high urease activity. The prebiotics described are oligosaccharides, inulin, lactulose and other vegetable fibres.

Dark fruit such as blackcurrant, cranberry and pomegranate are commonly consumed in many countries around the world. Epidemiological studies indicate that a high intake of fruit and vegetables may lower the risks of some chronic diseases (Kant et al., American Journal of Clinical Nutrition, 1993; Hertog et al., Lancet 342: 1007-1011, 1993; Lampe et al., American Journal of Clinical Nutrition, 1999). The fruit are rich in natural antioxidant polyphenolic compounds, such as anthocyanins, proanthocyanins and flavonols. They also contain simple phenols such as cinnamates and hydroxybenzoates and tannins such as ellagitannins, gallagitannins and condensed tannins. Some berry fruit and phenolic compounds within the berries have been reported to have antimicrobial effects (Puupponen-Pimia et al., Journal of Applied Microbiology 90: 494-507, 2001).

Blackcurrant juice is rich in vitamin C, and rich in anthocyanins such as cyanidin-rutinoside, cyanidin-glucoside, delphinidin-rutinoside, and delphinidin-glucoside. The blackcurrant anthocyanins have been found to be bioavailable to humans, but only a small percentage of the intake can be absorbed into the blood and excreted through urine (Rechner et al., Free Radical Research 36: 1229-1241, 2002). Therefore, the majority of ingested anthocyanin is retained in the small and large intestines during the digestion.and excretion processes.

Cranberry juice has been suggested to be beneficial in the prevention and treatment of urinary tract infections (Kontiokari et al., British Medical Journal 322: 1-5, 2001) due to its acidifying effects on urine as well as anti-microbial properties.

The consumption of pomegranate juice has been reported in a human study to be beneficial to cardiovascular health (Aviram et al., American Journal of Clinical Nutrition 71:1062-1076, 2000).

Dark fruit have not previously been described for other health promoting effects. Dark fruit selected from blackcurrant, cranberry, pomegganate or a mixture of two or more thereof have now been found to have a prebiotic effect. Furthermore, the selected dark fruit have been found to influence the pattern of the gut microflora, with a preferential promotion of the growth of 'beneficial' bacteria and concomitant reduction in the growth of 'harmful' bacteria.

The observed prebiotic effect thus differs from that of traditional prebiotics such as inulin, because dark fruit not only promote the growth of beneficial bacteria but also suppress the growth of harmful bacteria.

According to the present invention there is provided the use of dark fruit selected from blackcurrant, cranberry, pomegranate or a mixture of two or more thereof, for the manufacture of an orally ingestable composition for the promotion of the health of a mammal, characterised in that the dark fruit have a prebiotic effect.

Methods of improving the health of a mammal, preferably a human by the ingestion of the selected dark fruit are described. The methods will improve the health of a human by the oral consumption of dark fruit.

For the avoidance of doubt the term dark fruit includes the whole fruit, fruit pulp and fruit juice and mixtures thereof. The prebiotic effect of the selected dark fruit is suitably conferred by the consumption of dark fruit in the form of fruit juice.

In one aspect of the present invention the oral consumption of the selected dark fruit promotes the growth of beneficial gut bacteria.

In another aspect of the present invention the oral consumption of the selected dark fruit promote the growth of beneficial gut bacteria and reduces the growth of harmful bacteria.

In the context of the present invention dark fruit means fruit with black, red or blue skins selected from blackcurrant, cranberry and pomegranate, or a mixture of two or more thereof.

In the context of the present invention beneficial bacteria means those which do not excrete metabolic products that are detrimental to health. Some beneficial bacteria may produce compounds, like vitamins, which are positive to health. They may also be able to inhibit pathogens and are likely to be saccharolytic.

In the context of the present invention harmful bacteria means those which produce toxic products and may also have the ability to cause cell destruction by invasive capacities. Some toxic products produced by harmful bacteria may act locally in the gut and/or have systemic effects.

The invention may be applied to a wide range of compositions comprising the selected dark fruit. Compositions may be in the form of liquids, semi-solids or solids. Compositions may be dairy-based such as yoghurts. Compositions are suitably in the form of beverages. In the context of this invention, the term beverage encompasses ready to drink liquid forms as well as concentrates and powder formulations for dissolution. Ready to drink beverages may be still or carbonated.

The present invention is particularly suitable for use in ready to drink beverages with from about 1 to 40% (v/v) of single strength dark fruit juice, preferably from about 1 to 30% (v/v) and more preferably from about 1 to 15% (v/v) dark fruit juice.

The present inventors have found that the prebiotic effect of the selected dark fruit is particularly pronounced at low concentrations, from 1 to 20% (v/v) of single strength dark fruit juice.

The present invention is also particularly suitable for fermented and non-fermented dairy-based products such as yoghurts, fromage frais and milk based beverages.

Yoghurts will typically comprise from about 1-20% (v/v) fruit or fruit juice, preferably 1-10% (v/v) fruit or fruit juice.

Dairy-based beverages will typically comprise from about 1-40% (v/v) fruit juice.

For the avoidance of doubt single strength juice means the juice when pressed from mature fruit with neither water removed or added. Standards are set for a range of juices by Association of the Industry of Juices and Nectars from fruits and vegetables of the European Union (AIJN).

Compositions of the present invention may be unsweetened or sweetened with sugar or intense sweeteners such as saccharine, aspartyl phenyl alanyl methyl ester or other non-sugar sweeteners. Compositions may also contain other conventional additives such as flavourings, colourings, stabilisers etc.

Compositions according to the present invention may also contain probiotic and prebiotic mixtures.

Compositions according to the present invention may be prepared by mixing the ingredients according to conventional methods. Solids may be dissolved in water prior to mixing with other components. Typically beverage compositions are pasteurised prior to filling in bottles or cans or other packs or are "in-pack pasteurised" after filling.

### Testing for Prebiotic Effects with Faecal batch culture fermentation

Static batch cultures were set up in 100ml sterile bottles filled with 40ml pre-reduced sterile medium containing (g/l):

| **Ingredients** | **g/l** |
|---|---|
| peptone water | 2 |
| yeast extract | 2 |
| Na Cl | 0.1 |
| K₂HPO₄ | 0.04 |
| KH₂ PO₄ | 0.04 |
| Mg SO₄.7H₂O | 0.01 |
| CaCl₂.2 H₂O | 0.01 |
| Na HCO₃ | 2 |
| L-Cysteine HCl | 0.5 |
| Bile salts | 0.5 |
| | |
| Resazurin | 4ml |
| vitamin K₁ | 10µl |
| Tween 80 | 2ml |
| Hemin | 10ml. |

A 10% (w/v) faecal slurry from healthy donors was prepared using pre-reduced 0.1mol/l phosphate buffered saline (pH 7.0) and then mixed in a stomacher for 120 seconds. Each batch culture was inoculated with 5ml of the faecal slurry, to give a final concentration of 1% (v/v). 1 ml of L-Cysteine HCl was added to each batch culture to further reduce the medium. Batch cultures were maintained under a headspace of O₂-free N₂ and incubated at 37°C. Triplicate samples (1ml) were removed after 0, 5, 10 and 24 hours for enumeration of bacteria by FISH (Wang and Gibson, Journal of Applied Bacteriology 75: 373-380, 1993). The initial pH of the culture, once the faecal slurry was added, was 6.0. pH was not controlled during the incubation period.
Static batch culture fermentation was carried out in triplicate to determine the prebiotic capabilities of the fruit juices. Four different concentrations of each juice were tested as shown in tables 1, 2 and 3. The final concentrations were achieved by diluting juice concentrates according to the make up of the concentrates (Blackcurrant juice was 7.30 times concentrated while the cranberry and pomegranate juices were 8.82 and 6.98 times concentrate respectively). The growth of *Bifidobacterium* was assessed at 0, 10 and 24 hours of culturing. As a positive control with the study model, 1% w/v fructo-oligosaccharidcs was tested along with the juices. The experimental conditions were set at pH 6. The promotion of *Bifidobacterium* would be regarded as an indication of a prebiotic effect in this simple but efficient culturing model.

### Results

**Table 1 - Effects of blackcurrant juice on the growth of bifidobacteria in batch culture**

| **Blackcurrant juice (single strength)** | **N** | **0h** | **10 h** | **24 h** |
|---|---|---|---|---|
| 3.65% | 3 | 7.47±0.32 | 8.17±0.49* | 8.24±0.44* |
| 7.30% | 3 | 7.51±0.36 | 8.23±0.55* | 8.37±0.44** |
| 14.60% | 3 | 7.60±0.41 | 7.91±0.48* | 7.77±0.42* |
| 29.20% | 3 | 7.40±0.23 | 7.58±0.37 | 7.43±0.68 |

| | | | | |
|---|---|---|---|---|
| * p < 0.05; ** p < 0.01 compared with those measures at 0 h. N - number of samples taken in three | | | | |

**Table 2 - Effects of cranberry juice on the growth of bifidobacteria in batch culture**

| **Cranberry juice (single strength)** | **N** | **0 h** | **10 h** | **24 h** |
|---|---|---|---|---|
| 4.41% | 3 | 7.78±0.19 | 8.16±0.23 | 8.06±0.27 |
| 8.82% | 3 | 7.64±0.13 | 8.26±0.28* | 8.34±0.35* |
| 17.64% | 3 | 7.35±0.28 | 7.71±0.53 | 7.95±0.39* |
| 35.28% | 3 | 7.34±0.25 | 7.26±0.21 | 7.12±0.25 |

| | | | | |
|---|---|---|---|---|
| * p < 0.05, compared with those measures at 0 h. | | | | |

**Table 3 - Effects of pomegranate juice on the growth of bifidobacteria in batch culture**

| **Pomegranate juice (single strength)** | **N** | **0 h** | **10 h** | **24 h** |
|---|---|---|---|---|
| 3.49% | 3 | 7.49±0.24 | 8.11±0.37* | 8.04±0.38* |
| 6.98% | 3 | 7.50±0.29 | 8.42±0.36* | 8.28±0.51 |
| 13.96% | 3 | 7.68±0.16 | 8.38±0.40* | 8.32±0.55 |
| 27.92% | 3 | 7.44±0.28 | 8.19±0.30* | 8.09±0.56 |

| | | | | |
|---|---|---|---|---|
| * p < 0.05, compared with those measures at 0 h. | | | | |

### Testing for Prebiotic Effect with a model of the Human Colonic Microflora (Gut model design)

The continuous culture system consisted of 3 vessels, V1, V2 and V3. V1 had an operating volume of 280ml and the other two of 300ml. This *in vitro* model has been designed to mimic the three major regions of the human colon: proximal, transverse and distal colon respectively. Temperature (37°C) and pH were automatically controlled. Culture pH in the vessels was 5.5 in V1, 6.2 in V2 and 6.8 in V3. Each fermenter was magnetically stirred and the growth medium continuously sparged with oxygen-free nitrogen (15ml/min) and fed from a glass medium reservoir by a peristaltic pump to V1. V 1 sequentially supplied V2 and V3 via an overflow and a series of weirs. The overflow from V3 was led to a waste container.

The culture medium had the following constituents dissolved in distilled water (Macfarlane et al., FEMS Microbiology Ecology 26: 231-243, 1998):

| **Ingredient** | **g/l** |
|---|---|
| Starch (Sigma, Poole, Dorset,UK) | 5.0 |
| Pectin (citrus) | 2.0 |
| Guar gum | 1.0 |
| Mucin (porcine gastric type III) | 4.0 |
| Xylan (oatspelt) | 2.0 |
| Arabinogalactan (larch wood) | 2.0 |
| Inulin | 1.0 |
| Casein (Sigma, Poole, Dorset, UK) | 3.0 |
| Peptone water | 5.0 |
| Tryptone | 5.0 |
| Bile salts No3 | 0.4 |
| Yeast extract | 4.5 |
| NaCl | 4.5 |
| KCl | 4.5 |
| FeSO₄.7H₂O | 0.005 |
| KH₂PO₄ | 0.5 |
| MgSO₄.7H₂O | 1.25 |
| CaCl₂. 6H₂O | 0.1 |
| NaHCO₃ | 1.5 |
| L-Cysteine-HCl | 0.8 |
| Hemin | 0.05 |
| Tween 80 | 1.0 |
| | |
| Vitamin K | 10µl |
| Resazurin | 4ml |

The medium was autoclaved at 121°C for 15 minutes and placed under nitrogen gas whilst hot.

To inoculate the system, fresh faecal samples were obtained from healthy human subjects who reported no antibiotic usage during the previous 6 months and had no history of gastrointestinal disorder. A 20% (w/v) slurry was prepared using anaerobic phosphate buffered saline and approximately 100ml added to each culture vessel. The system was operated at a retention time (R) of 43h as calculated as the reciprocal of the dilution rate. The fermentation system was allowed to equilibrate overnight before the pump was started.

### Gut model dosing and sampling regime

Samples were taken at day -10 (before equilibration, ten day before dosing with juices), Day -2, -1 and 0 (to have an average of the last 3 days of the control period as baseline). After this period of 10 days, the medium was changed to 0.814% (w/v) carbohydrate (0.407% starch, 0.1628% Xylan, 0.1628% arabinogalactan, and 0.0814% inulin, all as w/v)) and fruit juice was added twice (every 12 hours- based on a flow rate from the reservoir of 20ml/h) daily. A concentration of 5% single strength juice was maintained in the flow through the system so that the initial dose of the concentrate into V1 was slightly higher. Samples were taken again at day 10, 11, 12 and then day 20, 21, 22.

### Results

The promotion of the growth of beneficial bacteria such as bifidobacteria and lactobacilli and/or inhibition of the growth of the deleterious bacteria such as bacteroides and clostridia would be regarded as a prebiotic effect. From the Tables 4-6, these effects have been clearly demonstrated.

Bacterial numbers present in the gut model treated with fruit juice concentrate are expressed as Log₁₀ cells/ml gut model +/- standard deviation. Mean values were generated from samples taken on three consecutive days.

**Table 4: Bacterial numbers in gut model dosed with blackcurrant juice concentrate**

| **Vessel** | **Total bacteria** | ***Bacteroides* spp.** | **Bifidobacteria** | ***C*. *histolytocum*** | **Lactobacilli** | ***E*. *rectale*** | ***E. limnosum*** |
|---|---|---|---|---|---|---|---|
| **V1** | | | | | | | |
| T0 | 9.62±0.08 | 8.90±0.15 | 8.20±0.06 | 7.49±0.07 | 7.63±0.10 | 8.89±0.10 | 6.98±0.08 |
| T10 | 9.77±0.07** | 7.72±0.03** | 8.68±0.05* | 7.13±0.08* | 7.65±0.12 | 9.18±0.04* | 7.31±0.05 |
| T20 | 9.98±0.16⁺⁺ | 6.98±0.10**⁺⁺ | 8.44±0.02* ⁺ | 7.10±0.11* | 7.78±0.04 | 9.17±0.08 | 7.64±0.20 |

| **V2** | | | | | | | |
|---|---|---|---|---|---|---|---|
| T0 | 9.54±0.07 | 8.46±0.07 | 8.03±0.09* | 7.23±0.10 | 7.62±0.05 | 8.73±0.09 | 7.33±0.13 |
| T10 | 9.54±0.08 | 8.47±0.08 | 8.48±0.03 | 6.93±0.07* | 7.62±0.11 | 9.02±0.03* | 7.78±0.06 |
| T20 | 9.76±0.11 | 8.45±0.09 | 8.36±0.09* | 6.69±0.11 | 7.84±0.05 | 9.12±0.10 | 7.55±0.01* |

| **V3** | | | | | | | |
|---|---|---|---|---|---|---|---|
| T0 | 9.59±0.07 | 8.51±0.06 | 8.34±0.04 | 7.26±0.20 | 7.45±0.05 | 8.77±0.06 | 7.07±0.06 |
| T10 | 9.64±0.07 | 8.31±0.09 | 8.57±0.13 | 7.11±0.20 | 7.56±0.11 | 8.98±0.06 | 7.42±0.12 |
| T20 | 9.64±0.07 | 8.13±0.15* | 8.45±0.02 | 6.36±0.09* ⁺ | 7.84±0.16 | 9.10±0.22 | 7.23±0.04 |

| | | | | | | | |
|---|---|---|---|---|---|---|---|
| *P < 0.05, ** P < 0.01 comparing values at T10 and T20 to those at T0 * P < 0.05, ⁺⁺ P < 0.01 comparing values T20 to those at T10 | | | | | | | |

**Table 5: Bacterial numbers in gut model dosed with cranberry juice concentrate.**

| **Vessel** | **Total bacteria** | ***Bacteroides* spp.** | **Bifidobacteria** | ***C*. *histolytocum*** | **Lactobacilli** | ***E*. *rectale*** | ***E. limnosum*** |
|---|---|---|---|---|---|---|---|
| **V1** | | | | | | | |
| T0 | 9.64±0.08 | 9.06±0.28 | 8.31±4.02 | 7.56±0.16 | 6.09±0.04 | 9.13±0.01 | 7.43±0.12 |
| T10 | 9.66±0.03 | 6.42±0.04** | 8.39±0.11 | 7.27±0.36 | 6.14±0.17 | 9.22±0.04 | 7.62±0.15 |
| T20 | 9.75±0.06* | 6.57±0.28** | 8.52±0.05* | 7.23±0.56 | 6.11±0.22 | 9.18±0.06 | 7.88±0.08⁺ |

| **V2** | | | | | | | |
|---|---|---|---|---|---|---|---|
| T0 | 9.50±0.08 | 8.86±0.06 | 8.12±0.18 | 7.58±0.07 | 6.39±0.07 | 9.08±0.06 | 7.36±0.15 |
| T10 | 9.67±0.05 | 8.45±0.04** | 8.41±0.09 | 6.87±0.37 | 6.12±0.37 | 9.13±0.24 | 7.55±0.19 |
| T20 | 9.71±0.05 | 8.60±0.04* | 8.47±0.05 | 7.08±0.31 | 6.40±0.31 | 9.14±0.15* | 7.80±0.12 |

| **V3** | | | | | | | |
|---|---|---|---|---|---|---|---|
| T0 | 9.32±0.07 | 8.54±0.09 | 8.02±0.1 | 7.56±0.04 | 6.64±0.13 | 8.97±0.07 | 7.19±0.20 |
| T10 | 9.73±0.06** | 8.43±0.10 | 8.41±0.113** | 6.74±0.18* | 6.40±0.15 | 9.09±0.05 | 7.61±0.07 |
| T20 | 9.64±0.05* | 8.50±0.09 | 8.53±0.04* | 6.83±0.18* | 6.76±0.14 | 9.11±0.06 | 7.79±0.09 |

| | | | | | | | |
|---|---|---|---|---|---|---|---|
| *P < 0.05, ** P < 0.01 comparing values at T10 and T20 to those at T0 ⁺ P < 0.05, ⁺⁺ P < 0.01 comparing values T20 to those at T10 | | | | | | | |

**Table 6: Bacterial numbers in gut model dosed with pomegranate juice concentrate.**

| **Vessel** | **Total bacteria** | ***Bacteroides* spp.** | **Bifidobacteria** | ***C*. *histolytocum*** | **Lactobacilli** | ***E*. *rectale*** | ***E. limnosum*** |
|---|---|---|---|---|---|---|---|
| **V1** | | | | | | | |
| T0 | 9.72±0.04 | 9.03±0.05 | 8.32±0.06 | 7.17±0.06 | 6.09±0.04 | 9.07±0.10 | 7.25±0.06 |
| T10 | 9.63±0.05 | 8.92±0.05* | 8.35±0.12 | 6.98±0.35 | 6.45±0.07* | 8.99±0.02 | 7.48±0.10 |
| T20 | 9.67±0.10 | 8.72±0.09 | 8.45±0.05** | 6.63±0.09* | 6.40±0.15 | 9.25±0.02⁺⁺ | 7.76±0.13⁺ |

| **V2** | | | | | | | |
|---|---|---|---|---|---|---|---|
| T0 | 9.39±0.02 | 8.85±0.04 | 8.32±0.05 | 6.96±0.14 | 6.37±0.01 | 8.97±0.14 | 7.25±0.06 |
| T10 | 9.50±0.07 | 8.80±0.11 | 8.34±0.12 | 6.72±0.25 | 6.41±0.13 | 9.13±0.03 | 7.62±0.12* |
| T20 | 9.56±0.05⁺ | 8.67±0.11 | B.70±0.05* ⁺ | 6.78±0.06 | 6.32±0.14 | 9.18±0.04 | 7.80±0.10* ⁺ |

| **V3** | | | | | | | |
|---|---|---|---|---|---|---|---|
| T0 | 9.60±0.03 | 8.67±0.04 | 8.37±0.02 | 6.95±0.07 | 6.30±0.08 | 9.01±0.09 | 7.22±0.06 |
| T10 | 9.49±0.15 | 8.76±0.02 | 8.42±0.07 | 6.61±0.19 | 6.48±0.09* | 8.98±0.07 | 7.58±0.10* |
| T20 | 9.58±0.03 | 8.67±0.04 | 8.77±0.05* ⁺ | 6.58±0.05** | 6.69±0.10* | 9.08±0.08 | 7.77±0.08** ⁺⁺ |

| | | | | | | | |
|---|---|---|---|---|---|---|---|
| *P < 0.05, ** P < 0.01 comparing values at T10 and T20 to those at T0 ⁺ P < 0.05, ⁺⁺ P < 0.01 comparing values T20 to those at T10 | | | | | | | |

The positive prebiotic effects observed in blackcurrant, cranberry, and pomegranate juices are illustrative of effects likely to be obtained with other dark fruit.

The invention is illustrated by way of the following non-limiting examples.

### Example 1 - Blackcurrant juice based drink

A drink containing blackcurrant juice 1-30% (v/v) single strength juice, optionally in combination with other juices such as apple juice, grape juice, or orange juice. The drink may also contain added ascorbic acid 0.27% w/w, calcium carbonate 0.24% w/w, aspartame 0.17% w/w, blackcurrant flavour 0.12% w/w, potassium sorbate 0.08% w/w, asesulfame K 0.06% w/w, sodium bisulphite 0.14% w/w, colour from grapeskin 0.00002% w/w, and water if this is not a 100% juice drink. The drink may also contain added carbohydrate e.g. glucose syrup. The drink may be still or carbonated.

### Example 2 - Cranberry juice based drink

A drink containing 1-40% v/v single strength juice of cranberry juice, optionally in combination with other juices such as apple juice or orange juice. The drink may also contain added carbohydrate e.g. sucrose 10% w/w, ascorbic acid 0.05%w/w, cranberry flavour 0.02% w/w, and preservatives e.g. potassium sorbate or sodium benzoate and water. The product may be still or carbonated.

### Example 3 - Pomegranate juice based drink

A drink containing 1-100% v/v single strength juice pomegranate juice, optionally in combination with other juices such as apple juice or orange juice. The drink may also contain added ascorbic acid 0.05% w/w, carbohydrate e.g. glucose or sucrose, aspartame or acesulfame K and water. The drink may be still or carbonated.

### Example 4 - Blackcurrant juice & dairy-based drink

A drink containing 1-20% v/v ssj of blackcurrant juice, optionally in combination with other juices such as apple juice or grape juice. The drink will contain dairy component(s) yoghurt 5% w/w and cream 2% w/w. The drink may also contain added carbohydrate e.g. sucrose 10% w/w, ascorbic acid 0.05% w/w, blackcurrant flavour 0.02% w/w, preservative potassium sorbate and water.

## Claims

1. The use of a dark fruit selected from blackcurrant, cranberry, pomegranate or a mixture of two or more thereof, for the manufacture of an orally ingestable composition for the promotion of health in a mammal **characterised in that** the dark fruit has a prebiotic effect.

2. Use as claimed in claim 1 wherein the dark fruit is in the form of fruit juice.

3. Use as claimed in claim1 to 2 wherein the composition is a beverage.

4. Use as claimed in claim 3wherein the composition is a beverage concentrate comprising 1-40% (v/v) single strength fruit juice.

5. Use as claimed in claim 3 wherein the composition is ready to drink beverage comprising 1-20% (v/v) single strength fruit juice.

6. Use as claimed in claim 1 wherein the composition further comprises a probiotic.

## Patentansprüche

1. Verwendung einer dunklen Frucht, die ausgewählt ist aus schwarzer Johannisbeere, Preiselbeere, Granatapfel oder einem Gemisch aus zwei oder mehreren dieser, zur Herstellung einer oral einnehmbaren Zusammensetzung zur Gesundheitsförderung in einem Säugetier, **dadurch gekennzeichnet, daß** die dunkle Frucht eine präbiotische Wirkung aufweist.

2. Verwendung gemäß Anspruch 1, worin die dunkle Frucht in der Form eines Fruchtsaftes ist.

3. Verwendung gemäß Anspruch 1 oder 2, worin die Zusammensetzung ein Getränk ist.

4. Verwendung gemäß Anspruch 3, worin die Zusammensetzung ein Getränkekonzentrat ist, das 1 bis 40 % (V/V) Direktfruchtsaft umfaßt.

5. Verwendung gemäß Anspruch 3, worin die Zusammensetzung ein trinkfertiges Getränk ist, das 1 bis 20 % (V/V) Direktfruchtsaft umfaßt.

6. Verwendung gemäß Anspruch 1, worin die Zusammensetzung ferner ein Probiotikum umfaßt.

## Revendications

1. Utilisation d'un fruit à peau foncée choisi parmi le cassis, la canneberge, la grenade ou un mélange de deux d'entre eux ou plus, pour la fabrication d'une composition pouvant être ingérée par voie buccale afin de promouvoir la santé chez un mammifère, **caractérisée en ce que** le fruit à peau foncée a un effet prébiotique.

2. Utilisation selon la revendication 1, dans laquelle le fruit à peau foncée est sous la forme d'un jus de fruit.

3. Utilisation selon la revendication 1 à 2, dans laquelle la composition est une boisson.

4. Utilisation selon la revendication 3, dans laquelle la composition est une boisson concentrée comprenant de 1 à 40 % (v/v) de jus de fruit de concentration naturelle, sans eau ajoutée ou éliminée.

5. Utilisation selon la revendication 3, dans laquelle la composition est une boisson prête à consommer comprenant de 1 à 20 % (v/v) de jus de fruit concentration naturelle, sans eau ajoutée ou éliminée.

6. Utilisation selon la revendication 1, dans laquelle la composition comprend, en outre, un probiotique.
